# FASCICULE DE BREVET EUROPEEN

(11) **EP 1 679 052 B1**
(45) Date de publication et mention de la délivrance du brevet: **12.08.2009**
(21) Numéro de dépôt: 05356165.0
(22) Date de dépôt: 22.09.2005
(51) Int. Cl.: A61F 5/01

(54) **Orthèse de genou rigide polycentrique à amplitude réglable**
Polyzentrische steife Knieorthese mit Amplitudenverstellung
Polycentric rigid knee brace with ajustable amplitude

(30) Priorité: 22.09.2004 FR 0410017
(43) Date de publication de la demande: 12.07.2006
(73) Titulaire: Gibaud, 42000 Saint Etienne (FR)
(72) Inventeur: Anquetil, Lionel, 42000 Saint Etiennne (FR)
(74) Mandataire: Delorme, Nicolas

(56) Documents cités:
- WO-A-86/04228
- DE-A1- 3 728 089
- DE-A1- 4 418 855
- US-A- 5 782 785

## Description

La présente invention concerne une orthèse de genou rigide destinée à être placée sur le genou d'un patient pour permettre de pallier une insuffisance fonctionnelle du genou.

L'immobilisation du genou peut être prescrite dans de nombreux cas, notamment entorse, immobilisation post-opératoire (rupture des ligaments croisés) ou laxité chronique de l'articulation nécessitant son immobilisation.

L'immobilisation du genou consiste essentiellement en une stabilisation de l'articulation dans le plan frontal et un contrôle de la flexion sur un secteur angulaire déterminé selon l'état de l'articulation dans le plan sagittal.

On connaît essentiellement deux familles d'orthèses. Il s'agit d'une part de genouillères en tissu élastique et, d'autre part, d'orthèses rigides comprenant des coques rigides.

En pratique, chacune de ces orthèses existe dans cinq à six tailles pour s'adapter à la morphologie de chaque patient. De plus, dans le cas d'orthèses rigides, elles doivent être disponibles pour appareiller des membres droits et des membres gauches.

Cela conduit donc à proposer des orthèses de genou en un grand nombre de références pour faire face à chaque morphologie.

L'invention a pour but de résoudre ces problèmes et propose une orthèse permettant de stabiliser une articulation du genou susceptible d'appareiller aussi bien un membre droit qu'un membre gauche et susceptible d'appareiller des patients de toutes morphologies.

De façon connue en soi, l'orthèse de genou rigide adaptable destinée à être placée autour d'un genou d'un patient comprend :
- une coque fémorale rigide hémicirculaire susceptible d'être mise en place sur la face antérieure d'une cuisse d'un patient s'étendant sensiblement sur la moitié de la circonférence de celle-ci, équipée d'au moins une sangle destinée à maintenir la coque fémorale sur la cuisse et de deux montants parallèles,
- une coque jambière rigide hémicirculaire susceptible d'être mise en place sur la face postérieure d'une jambe d'un patient s'étendant sensiblement sur la moitié de la circonférence de celle-ci, équipée d'au moins une sangle destinée à maintenir la coque jambière sur la jambe et de deux montants parallèles, la coque fémorale étant reliée à la coque jambière par deux articulations parallèles au plan sagittal postérieur reliant deux à deux les montants respectifs de la coque fémorale et de la coque jambière,

Selon l'invention, cette orthèse présente une symétrie générale par rapport au plan sagittal postérieur et la coque fémorale et la coque tibiale présentent chacune des moyens de réglage de la valeur de leur demi circonférence en fonction de la rotondité de la cuisse et de la jambe sur lesquelles l'orthèse est mise en place.

En outre, au moins l'une des sangles destinées à immobiliser la coque jambière supporte une barrette souple présentant deux nervures disposées en V pouvant venir en appui de part et d'autre de la tubérosité tibiale antérieure.

L'idée à la base de l'invention est de prévoir une orthèse de genou, ayant des coques rigides, qui est,
i. d'une part, symétrique par rapport au plan antéropostérieur pour l'appareillage d'un membre droit comme d'un membre gauche et,
ii. d'autre part, réglable au niveau de la géométrie de ses coques pour s'adapter à des membres de toute morphologie.

L'invention fournit, donc, une orthèse universelle dont le port peut être prescrit essentiellement dans des cas de problèmes ligamentaires (rupture ou entorse) du genou, cas dans lesquels il est traditionnellement prescrit l'appareillage par une orthèse spécifiquement adaptée aux mensurations du patient.

Selon une forme de réalisation préférée de l'invention, la coque fémorale est constituée de deux demi coques supportant chacune un montant, les deux demi coques étant chacune pourvue de moyens de liaison autorisant un recouvrement variable d'une coque par rapport à l'autre permettant de régler la demi circonférence de la coque fémorale en fonction de la rotondité de la cuisse.

Similairement, la coque tibiale est constituée de deux demi coques supportant chacune un montant, les deux demi coques étant chacune pourvue de moyens de liaison autorisant un recouvrement variable d'une coque par rapport à l'autre permettant de régler la demi circonférence de la coque tibiale en fonction de la rotondité de la jambe.

De préférence, pour chaque coque fémorale ou jambière, les moyens de liaison d'une demi coque sur l'autre sont constitués de deux panneaux de textile auto agrippant à boucles et crochets disposés en regard l'un de l'autre sur chacune des demi coques, les deux panneaux présentant un plage de recouvrement permettant de régler la demi circonférence de la coque.

Cette disposition permet un réglage extrêmement simple de la demi circonférence de chacune des coques.

Selon une forme de réalisation possible de l'invention, les deux liaisons reliant deux à deux les montants sont des articulations polycentriques permettant un réglage de l'amplitude angulaire.

L'invention prévoit que au moins l'une des sangles destinée à immobiliser la coque jambière supporte un barrette souple présentant deux nervures disposées en V pouvant venir en appui de part et d'autre de la tubérosité tibiale antérieure.

Selon d'autres dispositions possibles de l'orthèse de genou selon l'invention :
- la coque fémorale est équipée de deux sangles de fermeture.
- la coque tibiale est équipée de deux sangles de fermeture.
- la face intérieure de l'articulation polycentrique reçoit une pelote condylienne en mousse haute résilience.

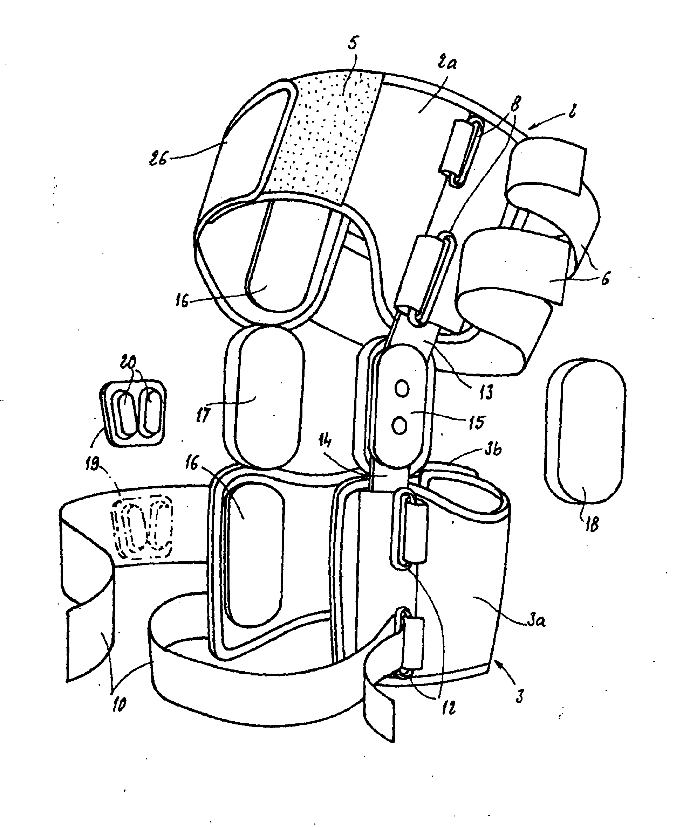

Pour sa bonne compréhension, l'invention est décrite en référence à la figure unique du dessin ci annexé représentant, à titre d'exemple non limitatif, une forme de réalisation d'une orthèse de genou selon celle-ci.

L'orthèse de genou, que l'on peut également qualifier de genouillère, présente une coque fémorale 2 et une coque jambière 3. Si l'on se réfère au dessin, on peut voir que la coque fémorale présente une forme hémicirculaire ouverte vers l'arrière et la coque jambière présente une forme hémicirculaire ouverte vers l'avant, de telle sorte que la coque fémorale s'engage sur la face antérieure de la cuisse et la cuisse jambière s'engage sur la face antérieure de la jambe.

De façon tout à fait spécifique à l'orthèse de genou objet de la présente invention, la coque fémorale 2 et la coque jambière 3 sont, chacune, constituées de deux demi coques dont les références sont respectivement 2a, 2b, 3a, 3b. Ces demi coques sont réalisées dans en matière plastique semi rigide et sont doublées sur leur face en contact du membre de matière textile.

Chacune des deux demi coques 2a, 2b, 3a, 3b est pourvue de moyens de liaison repositionnables ; en l'occurrence, il s'agit de deux panneaux de textile 5 auto agrippant à boucles et crochets collés en regard l'un de l'autre sur chacune des demi coques 2a, 2b, 3a, 3b, constituant respectivement les coques fémorales 2 et jambières 3 des panneaux présentant des dimensions telles qu'il se crée entre eux une large plage de recouvrement qui permet de fixer les deux demi coques de la coque jambière ou de la coque fémorale dans différentes positions permettant de régler la demi circonférence de la coque fémorale 2, ainsi que de la coque jambière 3.

Comme cela est représenté sur le dessin, la coque fémorale 2 est pourvue de deux sangles 6 qui sont fixées à l'une de ses extrémités et qui peuvent s'engager dans des anneaux 8 dont la seconde extrémité de la coque fémorale 2 est pourvue.

La coque jambière 3 présente une construction similaire dans la mesure où elle est également équipée de deux sangles 10 à l'une de ses extrémités, ces sangles 10 pouvant s'engager dans des anneaux 12 prévus à sa seconde extrémité.

La liaison entre la coque fémorale 2 et la coque jambière 3 est réalisée par deux montants 13 parallèles équipant la coque fémorale 2 et deux montants parallèles 14 équipant la coque jambière 3. Deux articulations 15 parallèles au plan sagittal relient deux à deux les montants respectifs de la coque fémorale 2 et de la coque jambière 3.

Dans l'exemple représenté, ces deux articulations 15 sont des articulations de type polycentrique connues en soi, c'est-à-dire présentant deux axes d'articulation, ce qui permet à l'orthèse d'avoir une cinématique plus proche de celle du genou que si elle était équipée d'une articulation monocentrique.

On note également que l'orthèse est équipée, sur sa face destinée à être en contact du patient, de capitonnage 16 antiglisse dans la coque fémorale 2 et dans la coque jambière 3, ainsi que de pelotes condyliennes 17 en mousse qui doublent la face interne de chaque articulation polycentrique. On peut voir également qu'un cache 18 recouvre la face extérieure des articulations polycentriques.

Une dernière caractéristique qu'il est important de noter est la présence d'une barrette 19 tibiale sur la sangle 10 supérieure de serrage de la coque jambière 3, (par supérieur, on entend la sangle la plus proche de l'articulation du genou). Cette barrette 19 tibiale, constituée de silicone, présente deux nervures 20 orientées selon un V dont la pointe est dirigée vers la sangle de serrage inférieure de la coque jambière.

Sur le dessin, pour des raisons de clarté d'illustration de cette barrette 19 tibiale, celle-ci est représentée en éclaté par rapport à l'orthèse elle-même.

Comme on peut donc le voir sur cette forme de réalisation de l'orthèse selon l'invention, celle-ci présente donc une coque fémorale 2 et une coque jambière 3 dont la demi circonférence est réglable et qui présente une symétrie par rapport au plan sagittal.

Il découle donc de ces caractéristiques spécifiques que cette orthèse peut être placée sur des sujets dont la rotondité de la jambe et de la cuisse est spécifique à chacun d'entre eux.

En d'autres termes, l'orthèse selon l'invention peut appareiller des patients de morphologies extrêmement diverses. On note également que sa symétrie par rapport au plan sagittal la destine à appareiller aussi bien des membres inférieurs droits que gauches.

La mise en place de cette orthèse se fait en réglant la demi circonférence de la coque fémorale 2 et de la coque jambière 3 en fonction de la rotondité spécifique de la cuisse et de la jambe du patient devant être appareillé.

On doit également insister sur la fonction de la barrette tibiale 19 qui participe à la conservation de la symétrie par rapport au plan sagittal puisque celle-ci vient se placer de part et d'autre de la tubérosité tibiale antérieure du patient. Cette barrette constitue également un excellent moyen de blocage de l'orthèse contre une possible rotation de l'orthèse.

L'orthèse, selon l'invention, présente donc les nombreux avantages indiqués précédemment puisqu'elle offre un réglage en fonction de la morphologie des quadriceps et du mollet du patient qu'elle doit appareiller, tout en réalisant bien entendu la fonction de stabilisation de la torsion du genou et d'autorisation de flexion selon une plage prédéterminée.

Bien entendu, l'invention n'est pas limitée à la forme de réalisation décrite ci-dessus à titre d'exemple non limitatif, mais elle en embrasse au contraire toutes les formes de réalisation. Ainsi, la fixation des demi coques l'une sur l'autre pourrait être réalisée par des boutons pression.

## Revendications

1. Orthèse de genou rigide adaptable destinée à être placée autour d'un genou d'un patient comprenant :
- une coque fémorale (2) rigide hémicirculaire susceptible d'être mise en place sur la face antérieure d'une cuisse d'un patient s'étendant sensiblement sur la moitié de la circonférence de celle-ci, équipée d'au moins une sangle (6) destinée à maintenir la coque fémorale (2) sur la cuisse et de deux montants (13) parallèles,
- une coque jambière (3) rigide hémicirculaire susceptible d'être mise en place sur la face postérieure d'une jambe d'un patient s'étendant sensiblement sur la moitié de la circonférence de celle-ci, équipée d'au moins une sangle (10) destinée à maintenir la coque jambière sur la jambe et de deux montants (14) parallèles, la coque fémorale (2) étant reliée à la coque jambière (3) par deux articulations (15) parallèles au plan sagittal reliant deux à deux les montants (13, 14) respectifs de la coque fémorale (2) et de la coque jambière (3),
**caractérisée en ce que** l'orthèse présente une symétrie générale par rapport au plan sagittal et la coque fémorale (2) et la coque jambière (3) présentent chacune des moyens de réglage de la valeur de leur demi circonférence en fonction de la rotondité de la cuisse et de la jambe sur lesquelles l'orthèse est mise en place et **en ce que**, au moins l'une des sangles (10) destinées à immobiliser la coque jambière (3) supporte une barrette (19) souple présentant deux nervures (20) disposées en V pouvant venir en appui de part et d'autre de la tubérosité tibiale antérieure.

2. Orthèse de genou selon la revendication 1, **caractérisée en ce que** la coque fémorale (2) est constituée de deux demi coques (2a, 2b) supportant chacune un montant (13), les deux demi coques (2a, 2b) étant chacune pourvue de moyens de liaison autorisant un recouvrement variable d'une demi coque par rapport à l'autre permettant de régler la demi circonférence de la coque fémorale (2) en fonction de la rotondité de la cuisse.

3. Orthèse de genou selon la revendication 1 ou la revendication 2, **caractérisée en ce que** la coque jambière (3) est constituée de deux demi coques (3a, 3b) supportant chacune un montant (14), les deux demi coques (3a, 3b) étant chacune pourvue de moyens de liaison autorisant un recouvrement variable d'une demi coque par rapport à l'autre permettant de régler la demi circonférence de la coque jambière (3) en fonction de la rotondité de la jambe.

4. Orthèse selon la revendication 2 ou la revendication 3, **caractérisée en ce que**, pour chaque coque fémorale (2) ou jambière (3), les moyens de liaison d'une demi coque sur l'autre sont constitués de deux panneaux (5) de textile auto agrippant à boucles et crochets disposés en regard l'un de l'autre sur chacune des demi coques les deux panneaux (5) présentant un plage de recouvrement permettant de régler la demi circonférence de chaque coque (2, 3).

5. Orthèse de genou selon l'une des revendications 1 à 4, **caractérisée en ce que** les deux liaisons (15) reliant deux à deux les montants sont des articulations polycentriques permettant un réglage de l'amplitude angulaire.

6. Orthèse selon l'une des revendications 1 à 5, **caractérisée en en ce que** la coque fémorale (2) est équipée de deux sangles (6) de fermeture.

7. Orthèse selon l'une des revendications 1 à 6, **caractérisée en ce que** la coque jambière (3) est équipée de deux sangles (10) de fermeture.

8. Orthèse selon l'une des revendications 1 à 7, **caractérisée en ce que** la face intérieure de l'articulation (15) reçoit une pelote condylienne (17) en mousse haute résilience.

## Claims

1. An adjustable rigid knee orthosis, to be placed around a patient's knee, comprising:
- a semicircular rigid femoral shell (2), which can be positioned on the front side of a patient's thigh, extending substantially over half of the circumference thereof, fitted with at least one bandage (6) for holding the femoral shell (2) on the thigh and with two parallel straps (13),
- a semicircular rigid leg shell (3), which can be positioned on the back side of a patient's leg, extending substantially over half of the circumference thereof, fitted with at least one bandage (10) for holding the leg shell on the leg and with two parallel straps (14), with the femoral shell (2) being connected to the leg shell (3) by two hinges (15) parallel to the sagittal plane connecting two by two the respective straps (13, 14) of the femoral shell (2) and of the leg shell (3),
**characterized in that** the orthosis has general symmetry with respect to the sagittal plane, and the femoral shell (2) and the leg shell (3) each have means for setting the value of the half circumference thereof depending on the roundness of the thigh and the leg on which the orthosis is positioned, and **in that** at least one of the bandages (10) for immobilizing the leg shell (3) supports a flexible web (19) having two ribs (20) arranged in V-shape capable of resting on either side of the anterior tibial tuberosity.

2. The knee orthosis according to claim 1, **characterized in that** the femoral shell (2) is composed of two half shells (2a, 2b) each supporting a strap (13), with both half shells (2a, 2b) being each provided with connecting means allowing for one half shell to be variably covered with respect to the other so that the half circumference of the femoral shell (2) can be adjusted depending on the roundness of the thigh.

3. The knee orthosis according to claim 1 or claim 2, **characterized in that** the leg shell (3) is composed of two half shells (3a, 3b) each supporting a strap (14), with both half shells (3a, 3b) being each provided with connecting means allowing for one half shell to be variably covered with respect to the other so that the half circumference of the leg shell (3) can be adjusted depending on the roundness of the leg.

4. The orthosis according to claim 2 or claim 3, **characterized in that** for each femoral (2) or leg (3) shell the means for connecting one half shell to the other are composed of two panels (5) of hook and loop textile with hooks and loops arranged opposite each other on each of the half shells, with both panels (5) having an overlap range so that the half circumference of each shell (2, 3) can be adjusted.

5. The knee orthosis according to any of claims 1 to 4, **characterized in that** the two links (15) connecting the straps two by two are polycentric hinges so that the angular amplitude can be adjusted.

6. The knee orthosis according to any of claims 1 to 5, **characterized in that** the femoral shell (2) is fitted with two closing bandages (6).

7. The knee orthosis according to any of claims 1 to 6, **characterized in that** the leg shell (3) is fitted with two closing bandages (10).

8. The knee orthosis according to any of claims 1 to 7, **characterized in that** the inner surface of the hinge (15) receives a condylar pad (17) of high resilience foam.

## Patentansprüche

1. Anpassungsfiähige steife Knieorthese, die dazu gedacht ist, um ein Knie eines Patienten herum angelegt zu werden, umfassend:
- eine halbkreisförmige steife Oberschenkelschale (2), die auf der Vorderseite eines Oberschenkels eines Patienten angebracht werden kann, sich im Wesentlichen über die Hälfte des Umfangs desselben erstreckt, mit mindestens einer Bandage (6), um die Oberschenkelschale (2) auf dem Oberschenkel zu halten, und mit zwei parallelen Stützen (13) ausgestattet ist,
- eine halbkreisförmige steife Beinschale (3), die auf der Rückseite eines Beins eines Patienten angebracht werden kann, sich im Wesentlichen über die Hälfte des Umfangs desselben erstreckt, mit mindestens einer Bandage (10), um die Beinschale am Bein zu halten, und mit zwei parallelen Stützen (14) ausgestattet ist, wobei die Oberschenkelschale (2) mit der Beinschale (3) über zwei Gelenke (15) parallel zur Sagittalebene verbunden ist, welche die jeweiligen Stützen (13, 14) der Oberschenkelschale (2) und der Beinschale (3) paarweise verbinden,
**dadurch gekennzeichnet, dass** die Orthese im Verhältnis zur Sagittalebene allgemein symmetrisch ist und die Oberschenkelschale (2) und die Beinschale (3) jeweils Mittel aufweisen, um den Wert ihres halben Umfangs je nach der Rundheit des Oberschenkels und des Beins, an denen die Orthese angelegt wird, einzustellen, und dass mindestens eine der Bandagen (10) zum Arretieren der Beinschale (3) eine flexible Strebe (19) mit zwei Rippen (20) trägt, die V-förmig angeordnet sind und auf beiden Seiten der vorderen Tuberosität des Schienbeins zur Anlage kommen können.

2. Knieorthese nach Anspruch 1, **dadurch gekennzeichnet, dass** die Oberschenkelschale (2) aus zwei halben Schalen (2a, 2b) besteht, die jeweils eine Stütze (13) tragen, wobei die beiden Halbschalen (2a, 2b) jeweils mit Verbindungsmitteln versehen sind, die eine variable Überdeckung einer Halbschale im Verhältnis zur anderen erlauben, wodurch es möglich ist, den halben Umfang der Oberschenkelschale (2) je nach Rundheit des Oberschenkels einzustellen.

3. Knieorthese nach Anspruch 1 oder 2, **dadurch gekennzeichnet, dass** die Beinschale (3) aus zwei Halbschalen (3a, 3b) besteht, die jeweils eine Stütze (14) tragen, wobei die beiden Halbschalen (3a, 3b) jeweils mit Verbindungsmitteln versehen sind, die eine variable Überdeckung einer Halbschale im Verhältnis zur anderen erlauben, wodurch es möglich ist, den halben Umfang der Beinschale (3) je nach Rundheit des Beins einzustellen.

4. Orthese nach Anspruch 2 oder 3, **dadurch gekennzeichnet, dass** für jede Oberschenkelschale (2) oder Beinschale (3) die Mittel zum Verbinden einer Halbschale mit der anderen aus zwei Platten (5) aus Klettstoff bestehen mit Haken und Ösen, die einander gegenüberliegend auf jeder der Halbschalen angeordnet sind, wobei die beiden Platten (5) einen Überdeckungsbereich aufweisen, wodurch der halbe Umfang jeder Schale (2, 3) einstellbar ist.

5. Knieorthese nach einem der Ansprüche 1 bis 4, **dadurch gekennzeichnet, dass** die beiden Verbindungen (15), welche die Stützen paarweise verbinden, polyzentrische Gelenke sind, wodurch die Winkelamplitude einstellbar ist.

6. Knieorthese nach einem der Ansprüche 1 bis 5, **dadurch gekennzeichnet, dass** die Oberschenkelschale (2) mit zwei Verschlussbandagen (6) ausgestattet ist.

7. Knieorthese nach einem der Ansprüche 1 bis 6, **dadurch gekennzeichnet, dass** die Beinschale (3) mit zwei Verschlussbandagen (10) ausgestattet ist.

8. Knieorthese nach einem der Ansprüche 1 bis 7, **dadurch gekennzeichnet, dass** die Innenseite des Gelenks (15) ein Kondylenpolster (17) aus hoch elastischem Schaumstoff aufnimmt.
